# EUROPÄISCHE PATENTSCHRIFT

(11) **EP 0 926 994 B1**
(45) Veröffentlichungstag und Bekanntmachung des Hinweises auf die Patenterteilung: **05.03.2003**
(21) Anmeldenummer: 98943762.9
(22) Anmeldetag: 21.07.1998
(51) Int. Cl.: A61B 17/28

(54) **CHIRURGISCHE FASS- UND HALTEZANGE**
SURGICAL GRASPING AND HOLDING PLIERS
PINCE CHIRURGICALE DE PREHENSION ET DE MAINTIEN

(30) Priorität: 22.07.1997 DE 19731454
(43) Veröffentlichungstag der Anmeldung: 07.07.1999
(73) Patentinhaber: Karl Storz GmbH & Co. KG, 78532 Tuttlingen (DE)
(72) Erfinder: BOCHE, Hartmut, D-88090 Immenstaad (DE); SCHERIEBLE, Hans, D-73734 Esslingen (DE)
(74) Vertreter: Weller, Wolfgang, Dr.rer.nat.
(86) Internationale Anmeldenummer: EP9804576
(87) Internationale Veröffentlichungsnummer: WO99004702

(56) Entgegenhaltungen:
- EP-A- 0 512 725
- DE-A- 4 216 971
- DE-U- 9 109 097
- US-A- 4 174 715
- US-A- 5 383 895

## Beschreibung

Die Erfindung betrifft eine chirurgische Faß- und Haltezange.

Chirurgische Faß- und Haltezangen dienen dazu, einen Gegenstand zwischen den Maulteilen zu erfassen und zu halten. Solche Gegenstände sind beispielsweise kugelförmige Mulltupfer, in diesem Fall spricht man dann von Tupferzangen. Bei zahlreichen laparoskopischen Eingriffen werden Präpariertupfer bzw. Stieltupfer benötigt. Mit deren Hilfe werden Gewebsflüssigkeiten bzw. Blut beseitigt. Ein Haupteinsatzgebiet besteht in der eigentlichen Präparationstechnik mit dem zwischen den Maulteilen eingespannten und gehaltenen Kugeltupfer. Durch schiebendes Dissezieren werden Gewebeschichten atraumatisch getrennt und somit Leitstrukturen während einer Operation dargestellt. In diesen Fällen arbeitet die Tupferzange zugleich als Präparierzange.

Andere Gegenstände, die in derartige Faß- und Haltezangen eingespannt sind, sind beispielsweise Nadeln zum Setzen einer Naht.

Die Gegenstände, also beispielsweise ein Kugeltupfer oder eine Nadel, werden durch die Instrumentierschwester bzw. den Instrumentierpfleger in die Haltezange eingespannt und dem Operateur angereicht. Dieser führt die Haltezange beispielsweise mit dem Tupfer durch einen Trokar ein und präpariert. Nach dem Gebrauch wird die Haltezange vom Trokar abgezogen und, wenn erforderlich, werden erneut kugelige Mulltupfer eingespannt. Entsprechendes gilt für das Setzen von chirurgischen Nähten.

Derartige Faß-, Halte- und Präparierzangen sind aus dem Katalog "Endoskopische Chirurgie, 2. Ausgabe, 1/94, Abschnitt 4, Blatt DG 5, Präparier- und Faßzangen" der Karl Storz GmbH & Co., Tuttlingen, Deutschland, bekannt.

Eine Arretierung der Griffteile in einer bestimmten Schwenkstellung zueinander und somit auch eine Arretierung der Maulteile in einer bestimmten Schließstellung wird über eine Raste bewerkstelligt. Die Raste ist als gekerbte Stange ausgebildet, die an einem der beiden Griffelemente angeordnet ist. Am anderen Griffelement sind entsprechende Vorsprünge vorhanden, die in die Kerbungen einrasten können. Eine Feder oder ein Hebel hält die Griffelemente in dem verrasteten bzw. arretierten Zustand. Dabei sind eine Vielzahl an Arretierstellungen möglich.

Im praktischen Einsatz derartiger chirurgischer Faß- und Haltezangen wurde nunmehr festgestellt, daß bei der Handhabung versucht wird, den zu haltenden Gegenstand, beispielsweise einen Kugeltupfer, möglichst fest in die Faßzange einzuspannen. Das bewegliche Griffelement ist ja als Hebel ausgebildet, wobei die Hebelachse die Scharnierachse darstellt, an der dieses bewegliche Griffelement am anderen Griffelement angelenkt ist. Der Abstand von der Scharnierachse zu der Stelle, an der das bewegliche Griffelement mit dem zu verschiebenden Betätigungselement verbunden ist, ist wesentlich kürzer als der Abstand von der Scharnierachse zu der am äußeren Ende des Griffelements angeordneten Fingerschlaufe, in die der Finger einer Hand, die die chirurgische Faß- und Haltezange ergreift, eingeschoben wird. Das Übersetzungsverhältnis beträgt etwa 10:1, das heißt, die Schließkraft einer Hand, etwa 10 kp, wird durch die Hebelwirkung auf das Zehnfache verstärkt, also auf etwa 100 kp.

Aufgrund der zuvor erwähnten Praxis, einen Kugeltupfer möglichst fest in die Faßzange einzuspannen, wirken über den Hebelmechanismus starke Kräfte auf die Maulteile ein, die dazu führen können, daß die Maulteile verbogen werden und/oder der Mechanismus beschädigt wird, so daß der Tupfer nicht mehr festgehalten wird und beim Präparieren verloren gehen kann. In diesem Fall muß im Körper der verlorene Tupfer gesucht und wieder ergriffen werden. Die Arretierung in Form der Zahnstangensperre steht ebenfalls unter der hohen Spannung dieses Haltedruckes, wodurch die Gefahr besteht, daß durch ungeschickte Handhabung die Raste ausrastet, wodurch die Arretierung gelöst wird und somit die Maulteile öffnen.

Ferner wurde im praktischen Einsatz festgestellt, daß die am proximalen Ende des Schaftes etwa rechtwinklig vom Schaft abstehenden Griffelemente die Freiheit des Operateurs stark einschränken, so daß zum Präparieren die Zange im Bereich des Übergangs von Schaft zum feststehenden Griffteil erfaßt wird. Die seitlich abstehenden Griffteile behindern oder stören den Operateur beim Präparieren. Die Raste, die sich quer zwischen den beiden Griffelementen erstreckt, kann bei solchen Präpariervorgängen versehentlich gelöst werden, so daß dann die von den Maulteilen gehaltenen Gegenstände, beispielsweise ein Kugeltupfer oder eine Nadel, im Körper verloren gehen können.

Die DE-A-4 216 971 zeigt eine Zange mit zwei Maulteilen zum Fassen und Halten von Gewebe oder dergleichen. Mindestens ein Maulteil ist relativ zum anderen durch axiale Verstellung einer Bestätigungsstange mittels einer Handhabe verschwenkbar. Die Schließstellung der Maulteile und der Handhabe ist durch eine Arretierung fixierbar, und die Schließkraft der Zangenmaulteile kann auf unterschiedliche Werte eingestellt werden.

Die EP-A-0 688 534 beschreibt ein chirurgisches Rohrschaftinstrument mit einem Schaft und einer darin verschieblich gelagerten Schub- und Zugstange zur Bewegung eines Werkzeuges am Ende des Schaftes und ein koaxial zum Schaft angeordnetes Griffteil am anderen Ende des Schaftes, in dem ein Handgriff um eine quer zur Schaftlängsachse verlaufenden Drehachse verschwenkbar gelagert ist, dessen Bewegung über Getriebemittel auf die Schub- und Zugstange übertragbar ist. Ferner ist ein federndes Rastelement vorgesehen, das bei der Bewegung des Handgriffs von einer Ausgangsstellung aus an einer Raste vorbei bewegt wird, dabei in diese eingreift und dadurch eine Rückbewegung des Handgriffs verhindert. Zur Vereinfachung der Konstruktion bildet das Rastelement und die Raste eine Umlaufsperre, bei der das Rastelement am Ende des Hubes des Handgriffs an der Raste vollständig vorbeibewegt ist und bei der Rückbewegung an der Rückseite der Raste vorbei in die Ausgangsstellung gelangt.

Aus der EP-A-0 512 725 ist ein medizinisches Instrument bekannt, bei dem ein Mechanismus zum Öffnen und Schließen der Maulteile durch Federkraft derart beaufschlagt ist, daß dadurch die Maulteile in Schließrichtung gedrückt werden. Durch ein Betätigungselement, das gegen die Kraft der Feder verschoben werden kann, kann ein Öffnen der Maulteile gesteuert werden. Wird das Betätigungselement freigegeben, wird es durch die Feder verschoben und dabei die Maulteile entsprechend der Kraft der Feder aufeinander gedrückt.

Aus der US-A-5 431 675 ist ein Verriegelungsmechanismus für ein medizinisches Instrument bekannt, bei dem ein Hebelgetriebe mit einer drehbaren Steuerscheibe in Verbindung steht. Durch außermittige Anordnung der Hebel an der Scheibe und entsprechende Hebelgelenkausbildung können Maulteile zwangsweise in eine ganz bestimmte Schließendstellung bewegt werden und halten dort das zwischen den Maulteilen vorhandene Teil mit einer bestimmten Haltekraft.

In der US-A-5 409 478 ist ein Griff eines medizinischen Instrumentes gezeigt, an dem ein seitlich abstehender Hebel angelenkt ist. Dieser Hebel steht über einen weiteren Hebel mit einem Betätigungselement zum Bewegen der Maulteile in Verbindung. Das Betätigungselement selbst wird über die Kraft einer Feder in einer Richtung gedrückt. Der seitlich abstehende angelenkte Hebel kann durch Handkraft an den Griff herangelegt werden, dabei wird das Betätigungselement über den zweiten Hebel gegen die Kraft der Feder verschoben. Dabei wird eine Raste überfahren, die dafür sorgt, daß beim Loslassen des angelenkten Hebels dieser nicht wieder ausspreizt.

Aus der US-A-5 383 895 ist ein als Faßzange ausgebildetes chirurgisches Instrument bekannt, dessen Betätigungselement, das zum Spreizen und Schließen der Maulteile vorgesehen ist, gegen die Kraft einer Feder hin- und herbewegt werden kann. Die Kraft der Feder beaufschlagt das Betätigungselement dahingehend, daß die Maulteile in Schließrichtung gedrückt werden.

Aus der US-A-5 211 655 ist eine medizinische Zange bekannt, die mit einer Arretierung versehen ist, über die die Maulteile in einer ganz bestimmten Stellung arretiert werden können.

US-A-4 174 715 zeigt eine chirurgische Faß- und Haltezange gemäß dem Oberbegriff von Anspruch 1.

Es ist Aufgabe der vorliegenden Erfindung, eine chirurgische Faß- und Haltezange zu schaffen, die ein sicheres Handhaben ermöglicht, und insbesondere keine Beschädigungen des erfaßten Teiles durch übermäßiges Aufwenden einer Haltekraft mehr möglich macht.

Erfindungsgemäß wird die Aufgabe durch eine chirurgische Faßund Haltezange gelöst, mit einem Schaft mit zumindest zwei Maulteilen, die am distalen Ende des Schaftes angeordnet sind, mit einem Griff, der am proximalen Ende des Schaftes angeordnet ist, mit einem mit dem Griff in Wirkverbindung stehenden Mechanismus zum Öffnen und Schließen der Maulteile, wobei der Mechanismus durch Federkraft derart beaufschlagt ist, daß dadurch die Maulteile in Schließrichtung gedrückt werden und durch Manipulation am Griff der Mechanismus gegen die Federkraft derart bewegbar ist, daß die Maulteile öffnen, und mit einer Arretierung zum Arretieren der Maulteile in einer bestimmten Stellung, wobei eine als starres Getriebe ausgebildete und in den Mechanismus eingreifende Vorrichtung vorgesehen ist, mittels derer die Maulteile zwangsweise in eine definierte Schließendstellung bewegbar sind, in der diese auf einen zwischen diesen aufgenommenen Gegenstand eine vorbestimmte Haltekraft ausüben, wobei die Vorrichtung einen Anschlag aufweist, der eine Überfahren der Schließendstellung auch bei erhöhtem Kraftaufwand sperrt, und wobei die Arretierung die Maulteile in dieser Schließendstellung arretiert.

Durch Vorsehen einer als starres Getriebe ausgebildten Vorrichtung, mittels der die Maulteile zwangsweise in eine definierte Schließendstellung bewegbar sind, werden unkontrollierte, über eine Schließendstellung hinausgehende Bewegungen ausgeschlossen. Dadurch werden Beschädigungen durch übermäßiges Anpressen der Maulteile aufeinander ausgeschlossen. Somit kann auch ausgeschlossen werden, daß beispielsweise ein Mulltupfer durch übermäßiges Einklemmen zwischen Maulteilen beschädigt oder gar in mehrere Teile aufgetrennt wird. Dies wird nun zusätzlich noch vorteilhaft dadurch gefördert, daß die Vorrichtung so ausgebildet ist, daß in der Schließendstellung auf den zwischen den Maulteilen aufgenommenen Gegenständen eine vorbestimmte Haltekraft ausgeübt wird. Wird mit einer erfindungsgemäßen Zange ein kugelförmiger Mulltupfer ergriffen, so wird dieser mit einer ganz bestimmten Haltekraft gehalten, die zum einen ausreichend ist, daß der Tupfer bei den Präpariervorgängen im Körper nicht verloren wird, andererseits aber auch sichergestellt ist, daß der Tupfer nicht durch übermäßige Haltekräfte beschädigt wird, so daß ausgeschlossen ist, daß sich der Mulltupfer beim Präparieren in mehrere Teile auflöst oder verloren wird.

Dadurch, daß die Vorrichtung einen Anschlag aufweist, der ein Überfahren der Schließendstellung auch bei erhöhtem Kraftaufwand sperrt, ist sichergestellt, daß auch bei besonders kraftvollem Zupacken die Maulteile nicht über die Schließendstellung hinaus bewegt werden können. Diese übermäßigen Kräfte werden dann von dem Anschlag der Vorrichtung aufgenommen, der entsprechend stabil oder massiv ausgebildet werden kann und muß nicht von den empfindlichen, bei sehr schlanken Geräten dementsprechend dünnen Bauelementen des Mechanismus zum Steuern der Maulteile aufgenommen werden. Die Vorrichtung arbeitet somit als ein "statischer Kraftbegrenzer".

Dadurch, daß die Arretierung so ausgebildet ist, daß die Maulteile gerade in der Schließendstellung arretiert werden, wird die Handhabbarkeit vereinfacht und die Betriebssicherheit erhöht. Bei den eingangs erwähnten Zangen mit der Zahnstangenraste waren ja zahlreiche Arretiermöglichkeiten vorhanden. Nunmehr ist eine bestimmte Arretiermöglichkeit vorhanden, nämlich dann, wenn die Maulteile die Schließendstellung erreicht haben. Es muß also nicht mehr von der Handhabungsperson entschieden werden, in welcher Schließstellung der Maulteile die Arretierung arretiert wird, sondern dies geschieht definiert in der Schließendstellung. Da mechanische Arretierungen meist mit einem typischen "Arretierungsklick" verbunden sind, weiß die Handhabungsperson, daß nun die Endstellung erreicht ist, so daß keine weitere Kraft auf den Griff ausgeübt werden muß. Der Steuerungsmechanismus der Vorrichtung zum Bewegen der Maulteile in die definierte Schließendstellung kann verschiedenartig ausgestaltet werden. Dieser steht mit den Maulteilen in Verbindung und bewegt diese definiert in die Schließendstellung.

Um im ersten Teil des Schließwinkelbereichs den Gegenstand aufzunehmen und vor dem Aufbringen der vorbestimmten Haltekraft zu halten, ist die Vorrichtung mit dem entsprechend ausgelegten Mechanismus verbunden. Das Wirkprinzip der vorliegenden Erfindung ergibt sich damit aus der Aufeinanderfolge der beiden folgenden Wirkungsbereiche:

### Wirkungsbereich des Mechanismus:

Aufnahme und Halten des Gegenstandes mittels Federkraft, d.h. Aufbringen einer "Vorhaltekraft" und axiale Bewegung des Mechanismus bis zum Eintritt in den Wirkungsbereich der Vorrichtung.

### Wirkungsbereich der Vorrichtung:

Aufbringen einer vorbestimmten Haltekraft und Arretierung.

Die Begrenzung der Kraft in der Handhabung chirurgischer Faßund Haltezangen erzeugt reproduzierbare Haltekräfte an den Maulteilen, was sich vorteilhaft unter Produkthaftungsaspekten auswirkt.

In einer besonders bevorzugten Ausgestaltung der Erfindung ist der Griff als sich in der Schaftachse längserstreckender Griff ausgebildet, und der Mechanismus und die Vorrichtung sind im Griff aufgenommen.

Diese Maßnahme hat nun den erheblichen Vorteil, daß eine besonders ergonomische Griffausgestaltung dadurch geschaffen wird, daß sich der gesamte Griff längs der Schaftachse erstreckt. Es sind in dieser Ausgestaltung keine seitlich abstehenden scherenartige Griffelemente mehr vorhanden, die den Operateur beim Handhaben, beispielsweise bei einem Präpariervorgang, stören. Daher kann die Zange an dem ergonomisch, grob stabförmigen Griff erfaßt werden und dadurch die chirurgische Faß- und Haltezange handhabungsfreundlich und handhabungssicher ergriffen und manipuliert werden.

In einer weiteren Ausgestaltung der Erfindung ist ein Abschnitt des Griffes um die Schaftachse drehbar, wobei dieser Abschnitt Teil der Vorrichtung ist, und wobei die Vorrichtung derart ausgestaltet ist, daß durch die Drehbewegung des Griffes die Maulteile in die Schließendstellung bewegbar sind.

Diese Maßnahme hat den Vorteil, daß die Vorrichtung und die durch diese erzeugte Schließbewegung der Maulteile nicht mehr durch Verschwenken eines scherenartigen beweglichen Griffteiles bewerkstelligt wird, sondern durch eine einfache Verdrehbewegung des beweglichen Griffabschnittes.

In einer weiteren Ausgestaltung der Erfindung ist im Griff eine Kulissenführung ausgebildet, über die die Drehbewegung des drehbaren Abschnittes des Griffes in eine axiale Verschiebebewegung eines Betätigungselementes des Mechanismus umsetzbar ist, wobei das Betätigungselement mit den Maulteilen in Verbindung steht.

Diese Maßnahme hat den Vorteil, daß durch mechanisch einfache und robuste Mittel die Drehbewegung des drehbaren Abschnittes des Griffes in die axiale Verschiebebewegung des Betätigungselementes zum Schließen der Maulteile umgesetzt wird.

In einer weiteren Ausgestaltung der Erfindung ist das Betätigungselement in einer Richtung gegen die Kraft einer Feder längs der Schaftachse verschiebbar, und ein Verschieben längs dieser Richtung erlaubt ein Öffnen der Maulteile in deren maximale Öffnungsstellung.

Diese Maßnahme hat nun den erheblichen handhabungsmäßigen Vorteil, daß das Öffnen der Maulteile, beispielsweise zum Einbringen eines Mulltupfers zwischen diese, das Betätigungselement lediglich gegen die Kraft der Feder etwas axial im Schaft verschoben werden muß. Dies kann einfach dadurch ausgeführt werden, daß das Betätigungselement proximal etwas über den Griff vorsteht und dieser auf diesen vorstehenden Bereich mit dem Daumen gedrückt wird und dadurch das Betätigungselement gegen die Kraft der Feder verschoben wird. Nachdem der Gegenstand, beispielsweise ein Mulltupfer, zwischen die maximal geöffneten Maulteile gebracht wurde, wird das Betätigungselement freigegeben, und die Feder verschiebt das Betätigungselement in die entgegengesetzte Richtung, wobei die Maulteile in Schließrichtung bewegt werden und auf den zwischen diesen erfaßten Gegenstand schon eine gewisse Haltekraft ausüben, die zumindest ausreichend ist, daß der Gegenstand nicht mehr von der Zange abfällt. Anschließend wird dann die Vorrichtung betätigt, mittels derer die Maulteile definiert in die Schließendstellung bewegt werden. Diese Aufteilung in zwei Teilschritte ist besonders handhabungsfreundlich und erlaubt im ersten Schritt bei den maximal geöffneten Maulteilen ein genaues Positionieren des Gegenstandes, beispielsweise des Mulltupfers zwischen den Maulteilen, anschließend im zweiten Schritt wird dann die definierte Haltekraft über die Vorrichtung in der Schließendstellung ausgeübt.

In einer weiteren Ausgestaltung der Erfindung ist das Betätigungselement mit einem Kulissenkörper verbunden, der in eine Kulisse eingreift, die dem drehbaren Abschnitt des Griffes zugehörig ist.

Diese Maßnahme hat den Vorteil, daß durch konstruktiv einfache und eine geringe Anzahl an Bauteilen eine kraftschlüssige Verbindung zwischen den Bauteilen geschaffen ist, die die Drehbewegung des Griffes in die entsprechende Linearbewegung des Betätigungselements bzw. dann in die Schließbewegung der Maulteile umsetzt. Solche mechanisch einfach aufgebauten Bauteile sind auch einfach zu reinigen bzw. zu sterilisieren, wodurch die Handhabungsfreundlichkeit und Betriebssicherheit weiter erhöht werden.

In einer weiteren Ausgestaltung der Erfindung ist die Kulisse als ein hohlzylindrischer drehbarer Abschnitt des Griffes ausgebildet, der den Kulissenkörper umgibt.

Diese Maßnahme hat den Vorteil, daß durch die Hohlzylindergeometrie eine Struktur vorgesehen ist, die auch übermäßig aufgewandte Drehkräfte gut aufnehmen kann, selbst bei einer sehr schlanken Bauweise der Zange.

In einer weiteren Ausgestaltung der Erfindung weist der Kulissenkörper zwei diametral gegenüberliegende, radial vorstehende Stifte auf, die in eine umfängliche, in axialer Richtung ansteigende Nut der Kulisse eingreifen.

Diese symmetrische Anordnung der doppelten Kulissenführung stellt eine besonders sichere und gute Kulissenführung dar, die auch in der Lage ist, übermäßig starke auf die Kulisse ausgeübte Kräfte gleichmäßig zu verteilen, ohne daß Deformierungen stattfinden können.

In einer weiteren Ausgestaltung der Erfindung weist jede ansteigende Nut einen sich axial erstreckenden Abschnitt auf.

Diese Maßnahme hat den Vorteil, daß über den sich axial erstreckenden Abschnitt der Kulissenkörper samt Betätigungselement axial verschoben werden kann, um in dem ersten Bewegungsabschnitt die Maulteile maximal spreizen zu können, damit der Gegenstand zwischen diese eingelegt werden kann. Nach Freigeben des Betätigungselementes bewegen sich die Maulteile aufgrund der Kraft der Feder in eine Schließstellung mit einer "Vorhaltekraft". An diesen sich axial erstreckenden Abschnitt schließt sich dann der in axialer Richtung ansteigende Abschnitt der Nut an, über die der Bewegungsvorgang der Vorrichtung zum Verschwenken der Maulteile in die Schließendstellung gesteuert wird.

In einer weiteren Ausgestaltung der Erfindung weist jede Nut einen Endabschnitt auf, der gegenüber dem ansteigenden Abschnitt etwas in axialer Richtung abfällt.

Diese Maßnahme hat nun den Vorteil, daß die Stifte, die ja von der Nut geführt werden, nach Überschreiten eines maximalen axialen Bewegungsweges in eine Art Hinterschneidung des abfallenden Abschnittes bewegt werden. Um die Maulteile zu öffnen, müssen die Stifte gegen die Kraft der Kulisse, die ausgehend vom Widerstand des von den Maulteilen gehaltenen Gegenstandes über die Maulteile und das Betätigungselement auf die Kulisse einwirkt, zunächst aus dem tiefsten Punkt des abfallenden Abschnitts über das Kurvenmaximum hinwegbewegt werden, wozu ein bestimmter Kraftaufwand notwendig ist. Damit ist ausgeschlossen, daß allein durch die Geometrie der ansteigenden Nut ein solches Rückstellmoment ausgeübt wird, daß sich die Maulteile von selbst öffnen. Der abfallende Endabschnitt der Nut stellt also schon eine Arretierung der Maulteile in der Schließendstellung dar.

In einer weiteren Ausgestaltung der Erfindung bildet ein geschlossenes Ende des Endabschnittes der Nut den Anschlag.

Diese Maßnahme hat den Vorteil, daß durch konstruktiv besonders einfache Mittel der Anschlag gebildet wird, der ein weiteres Drehen, also ein Überdrehen, sperrt. Übermäßige Kräfte können von dem geschlossenen Ende der Nut über die Kulisse abgeführt bzw. verteilt werden, ohne daß diese Kräfte auf die Maulteile einwirken.

In einer weiteren Ausgestaltung der Erfindung wirkt die Arretierung zwischen einem feststehenden Griffabschnitt und dem drehbaren Abschnitt derart, daß die Arretierung automatisch schließt, wenn die Stifte des Kulissenkörpers den Anschlag erreicht haben.

Diese Maßnahme hat nun den besonders handhabungsmäßigen Vorteil, daß genau dann, wenn die Stifte den Anschlag erreicht haben, die Arretierung schließt, somit genau in der gewünschten Position, das heißt in der Schließendstellung dann die Maulteile arretiert werden.

Dies ist deswegen besonders handhabungsfreundlich, da das bewegliche Griffteil lediglich so weit verdreht werden muß, bis die Handhabungsperson einen Widerstand spürt, nämlich wenn die Stifte des Kulissenkörpers das Ende der Nut erreicht haben und gerade in dieser Stellung die Arretierung automatisch schließt, was beispielsweise durch den typischen "Arretierungsklick" festgestellt werden kann, so daß dadurch die Handhabungsperson erkennt, daß die Schließendstellung erreicht worden ist.

In einer weiteren Ausgestaltung der Erfindung weist die Arretierung einen federbelasteten, sich axial erstreckenden Bolzen auf, der, wenn sich der verdrehbare Griffabschnitt in seiner Schließendstellung befindet, axial sperrend verfahrbar ist und eine Relativdrehung zwischen feststehendem Griffteil und drehbarem Griffteil sperrt.

Diese Maßnahme hat den konstruktiven Vorteil, daß sie durch mechanisch einfache Mittel zu bewerkstelligen ist, wobei der Bolzen ebenfalls dazu beitragen kann, übermäßige Drehkräfte über den gesamten Griff zu verteilen und abzuleiten.

In einer weiteren Ausgestaltung der Erfindung ist der Bolzen mit einem Schiebeelement verbunden, das von der Außenseite her bewegbar ist, jedoch der Außenkontur des Griffes angepaßt ist.

Diese Maßnahme hat zum einen den Vorteil, daß über die Zugänglichkeit von außen die Arretierung einfach gelöst werden kann. Dadurch, daß die Arretierung der Außenkontur des Griffes angepaßt ist, ist ausgeschlossen, daß beim Manipulieren diese Arretierung versehentlich gelöst wird, somit die Betriebssicherheit dadurch beachtlich erhöht ist.

In einer weiteren Ausgestaltung der Erfindung weist der Griff einen fest mit dem Schaft verbundenen, mit diesem koaxialen, zylindrischen Abschnitt auf, auf den der hohlzylindrische drehbare Abschnitt aufgeschoben ist, wobei der drehbare Abschnitt und der feststehende Abschnitt über eine lösbare Sicherung gegen axiales Abziehen gesichert sind.

Diese Maßnahme hat den Vorteil, daß durch konstruktiv einfache Maßnahmen der Griff montiert bzw. auch wieder demontiert werden kann, beispielsweise zu Reinigungs- und Sterilisierungszwecken.

In einer weiteren Ausgestaltung der Erfindung ist auf dem drehbaren Abschnitt eine Abschlußkappe fest montiert.

Diese Maßnahme hat den Vorteil, daß der drehbare Abschnitt, der ja als Kulisse arbeitet und im Nutbereich durchbrochen ist, durch die Abschlußkappe zur Außenseite hin abgeschlossen ist und somit keine Kontaminationen eindringen können. Die Abschlußkappe kann dann eine entsprechende ergonomische Außenkontur, beispielsweise Griffmulden oder dergleichen, aufweisen, über die die Drehbewegung ergonomisch und handhabungsfreundlich durchgeführt werden kann.

In einer weiteren Ausgestaltung der Erfindung sind zwei fassende Maulteile vorgesehen, deren Querschnittsprofil jeweils etwa halbovalförmig ist, und die geschlossenen Maulteile bilden etwa ein Oval.

Diese Maßnahme hat den Vorteil, daß nicht nur bei sehr schlanken Bauteilen eine günstige Geometrie vorliegt, um die Schließkräfte verformungsfrei auszuüben, sondern daß bei geschlossenen Maulteilen eine Geometrie resultiert, die ein hervorragendes Präparieren ermöglicht, somit die Zange als Präparierzange einsetzbar ist.

In einer weiteren Ausgestaltung der Erfindung weist ein Maulteil eine axial verlaufende V-Nut und das andere Maulteil einen in die V-Nut eingreifbaren Zahn auf.

Diese Maßnahme hat den Vorteil, daß radiale und axiale Kräfte, die auf den von den Maulteilen gehaltenen Gegenstand, z.B. einen Mulltupfer, beim Präparieren einwirken, von den Maulteilen über die Verzahnung aufgenommen werden, um den Verlust des Gegenstandes während des Einsatzes zu verhindern. Durch die V-förmige Nut in axialer Richtung werden seitliche Lasten aufgenommen, der Zahn im anderen Maulteil, der in die V-Nut eingreift, dient zur Aufnahme von axialen Lasten.

In einer alternativen Ausgestaltung weist die Vorrichtung zumindest einen am Griff angelenkten Hebel auf, dessen eines Ende mit dem Mechanismus zum Öffnen und Schließen der Maulteile in Eingriff steht, und dessen anderes Ende in der Offenstellung der Maulteile seitlich ausgeschwenkt ist, wobei das andere Ende zum Bewegen der Maulteile in die Schließendstellung D an den Griff heranverschwenkbar ist.

In dieser Alternative wird die definierte Bewegung in die Schließendstellung durch zumindest einen Hebel durchgeführt, was im Gegensatz zu der Lösung mit dem drehbaren Griffteil den Vorteil hat, daß durch entsprechende Ausgestaltung der Hebelgeometrie die notwendige Haltekraft durch die Bedienungsperson einfach aufgebracht werden kann.

In einer weiteren Ausgestaltung der Erfindung erstreckt sich der zumindest eine Hebel in der Schließendstellung D längs des langerstreckten Griffes.

Diese Maßnahme hat den Vorteil, daß der Hebel in der Schließendstellung nicht sperrig vom Griff absteht und die Bewegungsund Handhabungsfreiheit des Operateurs somit nicht stört.

In einer weiteren Ausgestaltung der Erfindung ist im Griff eine Längsnut ausgespart, in die der zumindest eine Hebel in der Schließendstellung der Maulteile passend aufnehmbar ist und die Kontur des Griffes nicht überragt.

In dieser Ausgestaltung schmiegt sich der angelegte Hebel voll an die Außenkontur des Griffes an, so daß keinerlei Beeinträchtigungen durch den Hebel bei der Handhabung in der Schließendstellung der Maulteile vorhanden ist.

In einer weiteren Ausgestaltung der Erfindung sind zwei diametral gegenüberliegend angeordnete Hebel vorgesehen.

Diese Maßnahme hat den Vorteil, daß die Kräfte zum Bewegen der Maulteile in die Schließendstellung symmetrisch durch die beiden Hebel auf den Mechanismus zum Öffnen und Schließen einwirken, wodurch die Gefahr von Verbiegen der Zange durch einseitig einwirkende Kräfte ausgeschlossen ist.

In einer weiteren Ausgestaltung der Erfindung ist die Arretierung als Schiebemuffe ausgebildet, die bei an den Griff herangeschwenkten Hebel über diese schiebbar ist und ein Ausschwenken sperrt.

Diese Maßnahme hat den Vorteil, daß die Arretierung in der Schließendstellung durch konstruktiv einfache und einfach zu handhabende Bauteile durchgeführt wird.

In einer weiteren Ausgestaltung der Erfindung ist die Schiebemuffe in der Schließendstellung über eine Kugelraste arretiert.

Diese Maßnahme hat den Vorteil, daß die Schiebemuffe, die die Hebel gegen Ausschwenken sperrt, durch die Kugelraste in einer ganz bestimmten Stellung noch zusätzlich arretiert wird.

In einer weiteren Ausgestaltung der Erfindung weist der Mechanismus zum Öffnen und Schließen der Maulteile ein an dem Schaft axial verschiebbares Betätigungselement auf, das derart mit dem einen Ende des Hebels in Verbindung steht, daß die Verschwenkbewegung des Hebels in eine axiale Verschiebbewegung des Betätigungselementes umsetzbar ist.

Mit dieser Ausbildung des Hebels als Knebelhebel können über mechanisch einfache und robuste Maßnahmen die Schwenkbewegungen der Hebel in die axiale Verschiebebewegung des Betätigungselementes im Schaft umgesetzt werden.

Es versteht sich, daß die vorstehend genannten und die nachstehend zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand eines ausgewählten Ausführungsbeispiels im Zusammenhang mit den beiliegenden Zeichnungen näher beschrieben und erläutert.

Es zeigen:
- Fig. 1: eine Seitenansicht einer chirurgischen Faß- und Haltezange mit maximal geöffneten Maulteilen, die Vorrichtung befindet sich in Position "offen",
- Fig. 2: eine der Fig. 1 entsprechende Seitenansicht in einer Zwischenstellung, in der die Maulteile einen Gegenstand, hier einen kugelförmigen Mulltupfer, bereits mit einer gewissen Vorhaltekraft halten,
- Fig. 3: eine den Fig. 1 und 2 entsprechende Seitenansicht, bei der die Maulteile in ihre Schließendstellung bewegt sind,
- Fig. 4: einen Längsschnitt des Griffes der Vorrichtung von Fig. 3 in einer Stellung, in der die Maulteile geschlossen sind,
- Fig. 5: eine abschnittsweise, teilweise freigelegte Seitenansicht des Griffes der Vorrichtung von Fig. 1, wobei vier Stellungen A, B, C und D der Maulteile angedeutet sind,
- Fig. 6: einen Schnitt längs der Linie VI-VI in Fig. 5,
- Fig. 7: einen Schnitt längs der Linie VII-VII in Fig. 5,
- Fig. 8: ein Kraft/Weg-Diagramm in Abhängigkeit von dem Verdrehwinkel des drehbaren Griffelementes, und
- Fig. 9: ein der Schnittdarstellung von Fig. 4 vergleichbarer Schnitt eines weiteren Ausführungsbeispiels mit seitlich ausschwenkbaren Hebeln.

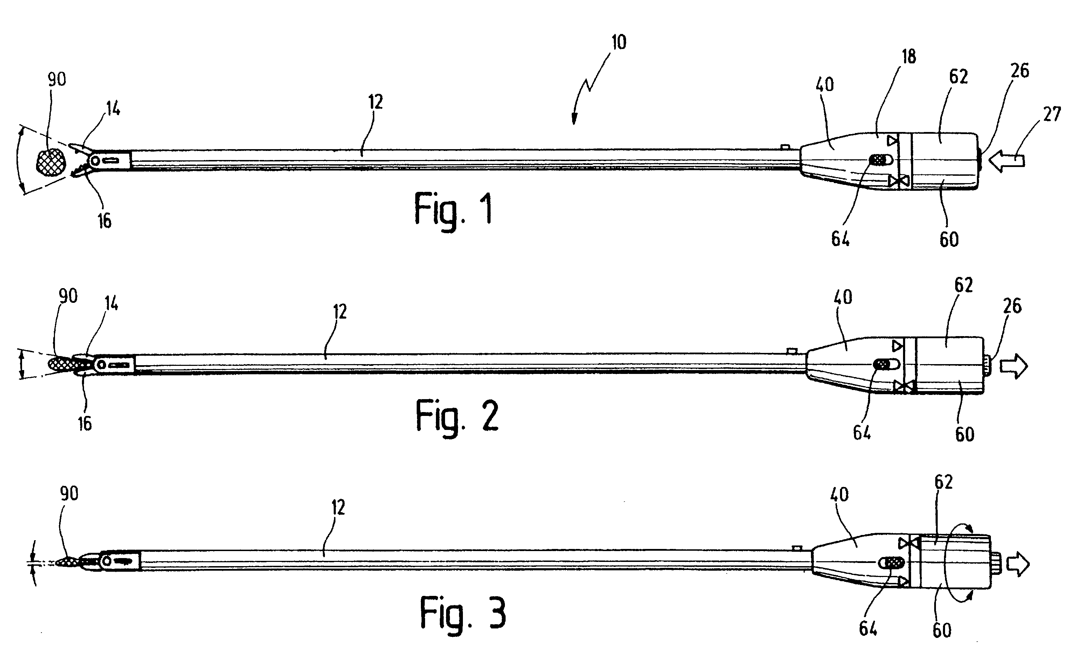

Eine in den Fig. 1 bis 3 dargestellte chirurgische Faß- und Haltezange ist in ihrer Gesamtheit mit der Bezugsziffer 10 versehen.

Die Zange 10 weist einen sich langerstreckten zylindrischen Schaft 12 auf, an dessen distalem Ende zwei Maulteile 14 und 16 angeordnet sind. Am proximalen Ende des Schaftes 12 ist ein sich in der Schaftachse längs erstreckender, etwa stabförmiger Griff 18 angeordnet.

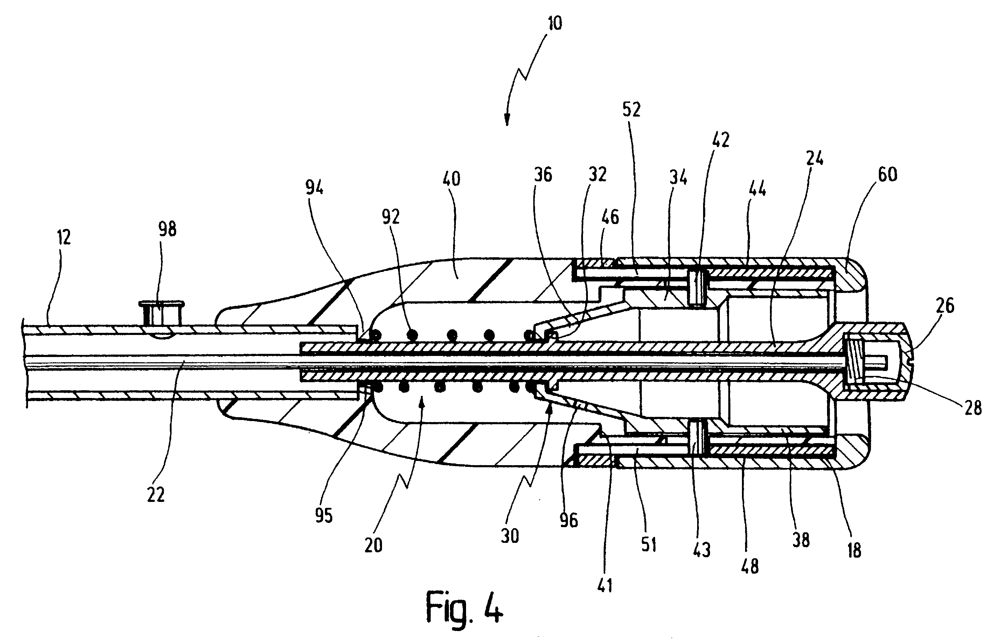

Im Griff 18 ist ein Mechanismus 20 zum Bewegen der Maulteile 14 und 16 aufgenommen, wie das insbesondere aus der Schnittdarstellung von Fig. 4 ersichtlich ist.

Der Mechanismus 20 weist ein stabförmiges Betätigungselement 22 auf, das durch den Schaft 12 hindurch reicht und am distalen Ende über ein hier nicht dargestelltes Kniehebelgestänge den beiden Maulteilen 14 und 16 verbunden ist, wie das an sich bei solchen Zangen bekannt ist. Am proximalen Ende reicht das Betätigungselement 22 etwas über das Ende des Griffes 18 hinaus, und über den Endbereich des Betätigungselementes 22 ist eine Drückerhülse 24 geschoben.

Die Drückerhülse 24 weist einen endseitigen Drücker 26 auf, und die Drückerhülse 24 erstreckt sich bis über einen inneren Flansch 94 im Griff 18 in Richtung Schaft 12.

Im Drücker 26 ist eine Stellschraube 28 angeordnet, die auf ein Gewinde des Betätigungselements 22 aufgeschraubt ist. Durch die Stellschraube 28 ist die relative Verschiebelage zwischen Drückerhülse 24 und Betätigungselement 22 in einem gewissen Bereich einstellbar und sichtbar.

Auf das Betätigungselement 22 ist von dessen distalem Ende her ein Kulissenkörper 34 geschoben, und zwar so weit, bis dieser an einem Ringflansch 32 des Betätigungselements 22 gelangt ist.

Der Kulissenkörper 34 weist einen konischen Abschnitt 36 auf, der sich proximal in einen zylindrischen Abschnitt 38 fortsetzt. Der Außendurchmesser des zylindrischen Abschnittes 38 des Kulissenkörpers 34 ist derart, daß dieser in etwa dem lichten Innendurchmesser eines zylindrischen Endabschnittes eines Gehäuses 40 entspricht, das fest mit dem Schaft 12 verbunden ist.

Der Abstand zwischen dem distalen Ende des zylindrischen Abschnitts 38 und einer Stufe 41 an der Innenseite des Gehäuses 40 ist so bemessen, daß das Öffnen der Maulteile durch Druck auf den Drücker 26 und entsprechenden Weg des Betätigungselementes 22 möglich ist.

Vom zylindrischen Abschnitt 38 des Kulissenkörpers 34 stehen diametral gegenüberliegend zwei radial vorspringende Stifte 42 und 43 vor.

Die Stifte 42 und 43 greifen in eine Kulisse 44 ein, die über den zylindrischen Endabschnitt des Gehäuses 40 geschoben ist.

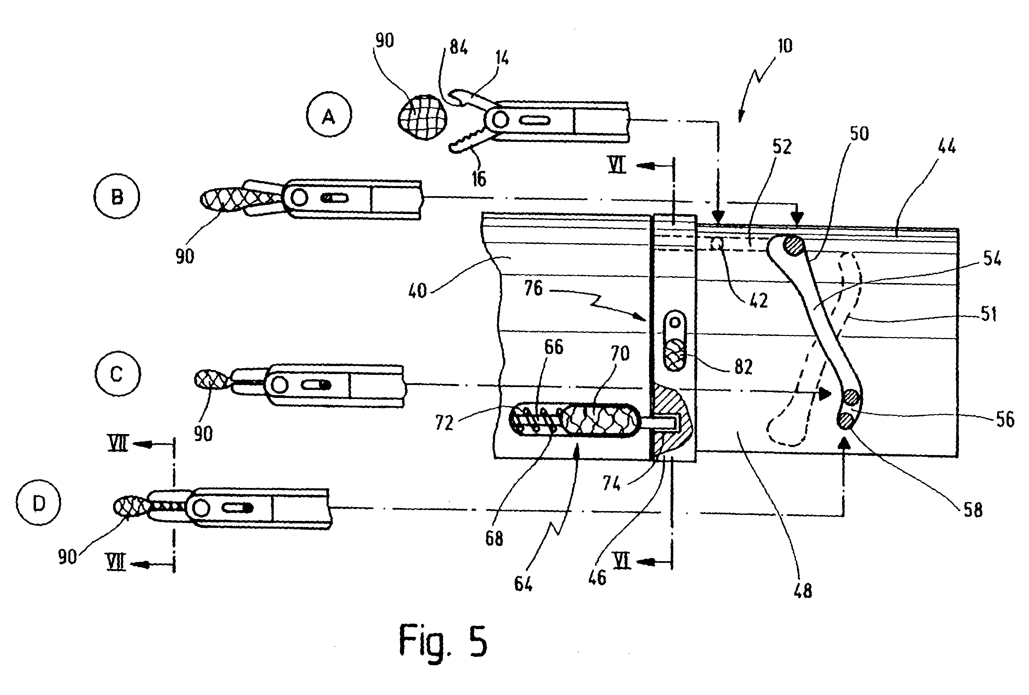

Eine Seitenansicht der Kulisse 44 ist der Fig. 5 zu entnehmen.

Die Kulisse 44 weist distal einen Ringflansch 46 auf, der auf einer Schulter an der Außenseite des Gehäuses 40 sitzt. Proximal weist die Kulisse 44 einen hohlzylindrischen Abschnitt 48 auf, der auf die Außenseite des zylindrischen Endabschnittes des Gehäuses 40 geschoben ist.

In dem hohlzylindrischen Abschnitt 48 der Kulisse 44 sind diametral gegenüberliegend zwei Nuten 50, 51 eingeschnitten. In die Nuten 50 bzw. 51 greifen die Stifte 42 bzw. 43 des Kulissenkörpers 34 ein. Dazu sind entsprechende Durchbrüche im Gehäuse 40 vorhanden. Die Nut 50 (und entsprechend drehsymmetrisch die Nut 51) weist einen axialen Abschnitt 52 auf, in den der Stift 42 des Kulissenkörpers 34 eingreift. Der axiale Abschnitt 52 öffnet in Richtung des Ringflansches 46 der Kulisse 44. Dadurch ist es zum einen möglich, daß nach Montieren des Kulissenkörpers 34 auf dem Betätigungselement 22 die Kulisse 44 von proximal her auf diesen aufgeschoben werden kann, darüber hinaus ist es möglich, daß sich der Zusammenbau aus Betätigungselement 22 und Kulissenkörper 34 axial längs des axialen Abschnittes 52 bewegen kann.

An das proximale Ende des axialen Abschnittes 52 der Nut 50 schließt sich ein umfänglicher, nach proximal ansteigender Abschnitt 54 an. Dieser ansteigende Abschnitt 54 endet in einem nach distal abfallenden umfänglichen Endabschnitt 56, dessen geschlossenes Ende einen Anschlag 58 bildet.

Der Übergang des axialen Abschnitts 52 in den umfänglichen, nach proximal ansteigenden Abschnitt 54 ist so gestaltet, daß die Einstellbarkeit über die Stellschraube 28 und die dadurch ausgelöste Änderung der relativen Verschiebelage zwischen Drückerhülse 24 und Betätigungselement 22 sowie auch eine unterschiedliche Widerstandskraft der von den Maulteilen zu haltenden Gegenstände, die zu unterschiedlichsten Teilöffnungswinkeln der Maulteile bei Aufbringung der Vorhaltekraft führen kann, aufgenommen werden können.

Über die Kulisse 44, wie sie in Fig. 5 ersichtlich ist, wird passend eine Abschlußkappe 60 zum Verschluß der Nuten mit entsprechend ergonomischer Ausbildung geschoben, wie sie in den Fig. 1 bis 4 ersichtlich ist. Die Abschlußkappe 60 sitzt dabei kraftschlüssig fest auf der Kulisse 44, der Zusammenbau aus Abschlußkappe 60 und Kulisse 44 bildet einen drehbaren Abschnitt 62 des Griffes 18.

Wie aus den Fig. 1 bis 3 und 5 zu ersehen, ist im Gehäuse 40 eine Arretierung 64 vorgesehen.

Die Arretierung 64 weist einen sich axial erstreckenden Bolzen 66 auf, der durch die Kraft einer Feder 68 derart beaufschlagt ist, daß er sich proximal Richtung Kulisse 44 bewegt. Der Bolzen 66 ist mit einem Schiebeelement 70 verbunden, das in einer Ausnehmung 72 im Gehäuse 40 angeordnet ist. Die Kontur des Schiebeelementes 70 ist derart, daß es nicht über das Gehäuse übersteht, dennoch von der Außenseite her mit einem Finger ergriffen und betätigt, also verschoben werden kann. Die Arretierung 64 ist so angeordnet, daß die Spitze des Bolzens 66 auf die ringförmige Stirnfläche des Ringflansches 46 gerichtet ist.

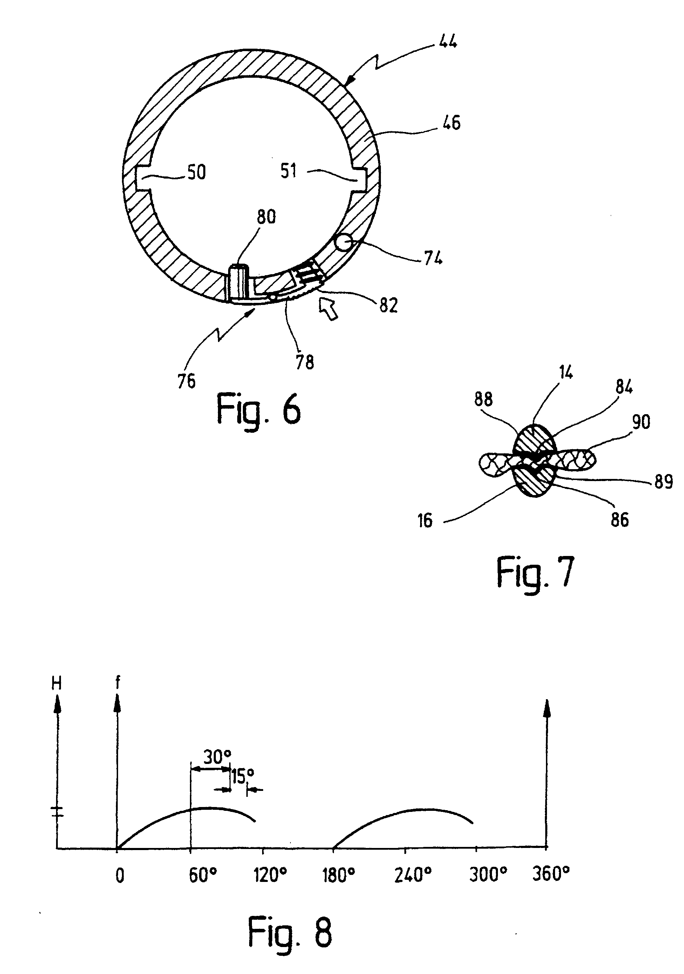

Im Ringflansch 46 ist, wie das aus der Schnittdarstellung von Fig. 6 dargestellt ist, eine Sacklochbohrung 74 vorgesehen, in die der Bolzen 66 einfahren kann. Dies geschieht in der in Fig. 5 dargestellten Drehstellung, die einer Schließendstellung D entspricht, wie das nachfolgend noch näher erläutert wird.

In dieser arretierten Position, wie sie in Fig. 5 ersichtlich ist, ist keine Relativdrehung zwischen Kulisse 44 und Gehäuse 40 mehr möglich.

Aus Fig. 5 und 6 ist ferner zu entnehmen, daß im Bereich des Ringflansches 46 der Kulisse 44 eine sich umfänglich erstreckende Sicherung 76 angeordnet ist, die axiales Abgleiten der Kulisse 44 von dem Gehäuse 40 hindert.

Die Sicherung 76 weist eine Wippe 78 auf, deren eines Ende mit einem Sperrbolzen 80 versehen ist. Der Sperrbolzen 80 greift in eine umfängliche Ausnehmung an der Außenseite des Gehäuses 40 ein, erlaubt somit die Relativdrehung zwischen Kulisse 44 und dem Gehäuse 40 um die Schaftachse, sperrt jedoch vor einem axialen Abgleiten. Die Länge der umfänglichen Aussparung ist so bemessen, daß bei Drehung des Griffes 18 die Schließendstellung erreicht wird.

Die Wippe 78 weist auf der dem Sperrbolzen 80 gegenüberliegenden Seite eine Drucktaste 82 auf. Durch Drücken der Drucktaste 82 rastet der Sperrbolzen 80 aus, und die Kulisse 44 kann proximal vom Gehäuse 40 abgezogen werden, beispielsweise zu Reinigungs- und Sterilisierungszwecken.

Im endmontierten Zustand, wie er in Fig. 4 dargestellt ist, ist zwischen Flansch 94 des Gehäuses 40 und dem auf das Betätigungselement 42 aufgeschobenen Kulissenkörper 34 eine Schraubenfeder 92 gelegt, die so vorgespannt ist, daß der Zusammenbau aus Betätigungselement 22 und Kulissenkörper 34 nach proximal gedrückt wird. In der in Fig. 4 dargestellten Stellung sind die Maulteile 14 und 16 durch die Kraft der Feder 92 geschlossen.

Die Zange 10 wird so gehandhabt, daß der Griff 18 mit einer Hand ergriffen wird und mit einem Daumen auf den Drücker 26 gedrückt wird, wie das in Fig. 1 durch einen Pfeil 27 dargestellt ist. Der drehbare Teil des Griffes 18 befindet sich dabei in der Position, in der die Vorrichtung "geöffnet" ist, d.h. der Stift 42 sich im axialen Abschnitt 52 der Nut 50 befindet. Dadurch wird das Betätigungselement 22 gegen die Kraft der Feder 92 in Richtung des distalen Endes verschoben, und die Maulteile 14 und 16 werden maximal gespreizt, beispielsweise auf einen Öffnungswinkel von ca. 50°.

Diese Eindrückbewegung wird dadurch begrenzt, daß der Kulissenkörper 34 auf eine Stufe 41 bzw. den Anschlag an der Innenseite des Gehäuses 40 trifft. Diese Öffnungsstellung ist in Fig. 1 bzw. in Fig. 5 mit der Bezeichnung A versehen. Der Stift 42 wurde aus der Stellung von Fig. 4 längs des axialen Abschnittes 52 der Nut 50 verschoben und hat die in Fig. 5 durch den Pfeil ausgehend von der Stellung A markierte Position erreicht. In dieser Stellung kann nun ein Gegenstand, beispielsweise ein Kugeltupfer 90 aus Mullmaterial, zwischen die Maulteile 14 und 16 gebracht werden. Am Maulteil 14 ist zumindest ein Zahn 84 vorgesehen, der in eine entsprechende Lücke 86 am anderen Maulteil 16 eingreifen kann, wie das auch aus der Schnittdarstellung von Fig. 7 ersichtlich ist.

Nachdem der Kugeltupfer 90 zwischen die Maulteile 14 und 16 verbracht wurde, wird der Drücker 26 freigegeben, und die vorgespannte Feder 92 verschiebt den Zusammenbau aus Betätigungselement 22 und Kulissenkörper 34 in Richtung proximalem Ende, wodurch die Maulteile 14 und 16 etwas geschlossen werden, bis sie die in Fig. 5 mit B bezeichnete Stellung erreicht haben. In dieser teilweise geschlossenen Stellung ist der Kugeltupfer 90 zwischen den Maulteilen 14 und 16 gefangen und wird mit einer Vorhaltekraft aufgrund der Kraft der Feder 92 gehalten.

Der Stift 42 hat sich nun längs des axialen Abschnittes 52 der Nut 50 in die in Fig. 5 mit dem Pfeil ausgehend von der Stellung B angezeigten Position bewegt. In dieser Position befindet sich der Stift 42 auch am Anfang des ansteigenden Abschnittes 54 der Nut 50. Der Übergangsbereich des axialen Abschnittes 52 in den umfänglichen und axial ansteigenden Abschnitt 54 der Nut 50 ist so ausgebildet, daß eine Änderung der relativen Verschiebelage zwischen der Drückerhülse 24 und dem Betätigungselement 22 sowie auch eine unterschiedliche Widerstandskraft der von den Maulteilen zu haltenden Gegenstände, die zu unterschiedlichen Teilöffnungswinkeln der Maulteile bei Aufbringung der Vorhaltekraft führen kann, aufgenommen werden können.

Nunmehr wird der Zusammenbau aus Abschlußkappe 60 und Kulisse 44, die den drehbaren Abschnitt 62 des Griffes 18 bilden, um die Schaftachse verdreht, wie das aus dem Übergang von Fig. 2 zu Fig. 3 ersichtlich ist.

Dabei bewegt sich der Stift 42 zunächst längs des ansteigenden Abschnittes 54 der Nut 50 und anschließend in den abfallenden Endabschnitt 56 hinein, bis der Anschlag 58 erreicht ist.

In Fig. 5 bezeichnet der Buchstabe C die Zwischenstellung, in der der Stift 42 gerade den Übergang von dem ansteigenden Abschnitt 54 zum abfallenden Endabschnitt 56 erreicht hat. In diesem Zustand ist das Betätigungselement 22 samt Kulissenkörper 34 maximal gegen die Widerstandskraft des von den Maulteilen gehaltenen Gegenstandes nach proximal bewegt. Anschließend bewegt sich dieser Zusammenbau wieder geringfügig zurück Richtung distalem Ende, bis dann die Schließendstellung D erreicht wird.

Die zusammenwirkenden Bauteile, Kulisse 44, Kulissenkörper 34 und mit diesem verbundenes Betätigungselement 22 bilden eine als starres Getriebe arbeitende Vorrichtung 30, um die Maulteile 14 und 16 zwangsweise in die definierte Schließendstellung D zu bewegen.

In dieser Schließendstellung D, wie sie in Fig. 5 und auch in Fig. 3 ersichtlich ist, wird der Kugeltupfer 90 mit einer vorbestimmten Kraft zwischen den Maulteilen 14 und 16 gehalten.

Aus der Schnittdarstellung von Fig. 7 ist ersichtlich, daß die Maulteile 14 und 16 jeweils ein Querschnittsprofil eines Halbovals 88 bzw. 89 haben. Daraus resultiert bei den in der Schließendstellung D geschlossenen Maulteilen eine insgesamt etwa ovalförmige Außenkontur, die ein Präparieren mit den geschlossenen Maulteilen 14 und 16 erlaubt.

In der Schließendstellung D wurde die Kulisse 44 so verdreht, daß deren Sacklochbohrung 74 vor dem Bolzen 66 der Arretierung 64 zum Liegen kommt, so daß nunmehr die Feder 68 den Bolzen 66 in das Sackloch einschiebt und die Arretierung 64 schließt. Nunmehr kann die Zange 10 beispielsweise über einen Trokar in einen Körper eingeführt werden, und es können entsprechende Präpariervorgänge durchgeführt werden.

Nach Abziehen vom Trokar wird mittels des Schiebeelementes 70 der Bolzen 66 gegen die Kraft der Feder 68 verschoben und die Arretierung 64 gelöst. Anschließend wird der drehbare Abschnitt 62 entgegen der zuvor beschriebenen Richtung gedreht, wodurch die Maulteile 14 und 16 teilweise wieder geöffnet werden, also wieder die Stellung wie sie in Fig. 2 dargestellt ist erreicht haben. Anschließend wird auf den Drücker 26 gedrückt, um die Maulteile 14 und 16 maximal zu spreizen, so daß der Kugeltupfer 90 entnommen werden kann und beispielsweise durch einen anderen neuen ersetzt werden kann.

In Fig. 8 ist ein Diagramm dargestellt, das die Kraft f bzw. den Hub H des Betätigungselementes 22 in Abhängigkeit des Verdrehwinkels des drehbaren Abschnittes 62 des Griffes 18 darstellt. Aufgrund der sehr kleinen Wege des Betätigungselementes 22 ergibt sich ein relativ flacher Kurvenverlauf mit entsprechendem Drehmoment, das heißt, es erfolgt eine relativ gleichmäßige kraftbeaufschlagte Drehbewegung, die nach Erreichen des Anschlages gesperrt ist. Der Bedienungsperson zeigt das Verschieben des Schiebeelementes 70 der Arretierung 64 an, daß der verdrehbare Abschnitt 62 in die richtige Stellung gedreht worden ist und nunmehr die Maulteile 14 und 16 mit der gewünschten Haltekraft beaufschlagt sind. Eine weitere Krafteinwirkung auf den drehbaren Abschnitt 62 verursacht keine weitere Bewegung der Maulteile 14 und 16 mehr. Die aufgewandten Kräfte werden anschließend auf den Griff 18 abgeleitet. Die Position entspricht einer Stellung "geschlossen" bzw. "on". Am Griff können diese Stellungen z.B. durch die Worte "on" und "off" bezeichnet werden.

In Fig. 4 bezeichnen die Bezugsziffern 95 und 96 Spülbohrungen im Flansch 94 bzw. in dem konischen Abschnitt 36 des Kulissenkörpers, so daß der gesamte Innenraum zum Reinigen gespült werden kann. Dazu ist am Schaft 12 ein seitlich vorspringender Stutzen 98 vorgesehen, über den eine Spül- oder Reinigungsflüssigkeit zugeführt und durch den Griff über die Reinigungsöffnungen 95 und 96 hindurchgeführt werden kann.

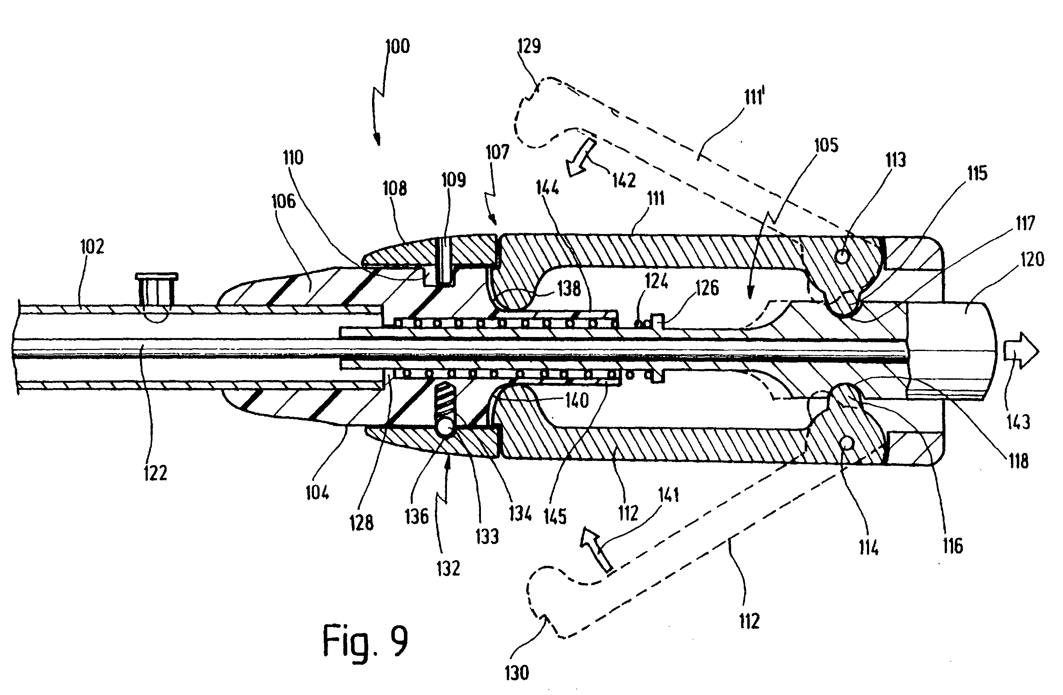

Bei einem in Fig. 9 dargestellten weiteren Ausführungsbeispiel ist eine Zange 100 lediglich im Bereich ihres Griffes 104 dargestellt.

Auch die Zange 100 weist am distalen Ende zwei Maulteile auf, die an einem Schaft 102 montiert sind.

Ein durch den Schaft 102 hindurchreichendes Betätigungselement 122 ist wie zuvor beschrieben endseitig mit einer Drückerhülse 120 versehen.

Zwischen einem nach innen vorspringenden Flansch 128 eines Gehäuses 106 und einem entsprechenden äußeren Ringflansch 126 an der Drückerhülse 120 erstreckt sich eine Feder 124.

Im Gehäuse 106, das fest mit dem Schaft 102 verbunden ist, sind diametral gegenüberliegend in zwei im Gehäuse 106 ausgesparten Längsnuten 138 bzw. 140 zwei Hebel 111, 112 angeordnet. Die Hebel 111 bzw. 112 sind jeweils um eine Achse 113 bzw. 114 verschwenkbar. Zwei kurze Hebelarme jedes Hebels 111, 112 jenseits der Achse 113, 114 sind als Knebel 115 bzw. 116 ausgebildet, die in entsprechende Aussparungen 117 bzw. 118 an der Außenseite der Drückerhülse 120 eingreifen.

In der Schnittdarstellung von Fig. 9 sind in durchgezogenen Linien die beiden Hebel 111, 112 in ihrer eingeschwenkten bzw. angelegten Position dargestellt, diese Position entspricht der Schließendstellung D der Maulteile, wie zuvor beschrieben. Zur Arretierung der Hebel 111, 112 in dieser Lage ist an diesen jeweils eine Stufe 129, 130 vorgesehen, auf die eine Arretierung 107 in Form einer Schiebemuffe 108 geschoben werden kann, wie das in der Schnittdarstellung von Fig. 9 dargestellt ist. Die Schiebemuffe 108 ist mit einem radial nach innen vorspringenden Zapfen 109 versehen, der in einer entsprechenden Aussparung 110 an der Außenseite des Gehäuses 106 aufgenommen ist. Die Aussparung 110 begrenzt den axialen Verschiebeweg der Schiebemuffe 108.

In der in der Schnittdarstellung von Fig. 9 dargestellten sperrenden Verschiebestellung ist die Schiebemuffe 108 zusätzlich über eine Kugelraste 132 gesichert. In einer dem Zapfen 109 etwa diametral gegenüberliegenden Sacklochbohrung ist eine Feder 134 aufgenommen, die radial außen auf einer Kugel 133 ruht. Die Feder 134 drückt die Kugel 133 in eine entsprechende Ausnehmung 136 an der Innenseite der Schiebemuffe 108.

Wird die Schiebemuffe 108 unter Überwindung der Sperrkraft der Kugelraste 132 nach distal verschoben, tritt die Schiebemuffe 108 aus den Stufen 129 bzw. 130 der Hebel 111 bzw. 112 aus, und diese können um ihre entsprechenden Achsen 113 bzw. 114 nach außen verschwenkt werden.

Eine solche Stellung ist in Fig. 9 durch die gestrichelte Linie angedeutet. In dieser Stellung haben die Knebel 115, 116 die Drückerhülse 120 ebenfalls nach distal verschoben, dadurch wurden die Maulteile geöffnet. Durch weiteres Drücken auf die Drückerhülse 120 können die Maulteile ggf. noch weiter geöffnet, somit auch die Hebel 111, 112 noch weiter gespreizt werden.

Der Zusammenbau aus Hebel 111, 112 und Drückerhülse 120 bildet somit die als starres Getriebe arbeitende Vorrichtung 105 zum zwangsweise und definierten Bewegen der Maulteile in die zuvor beschriebene Schließendstellung D.

Dazu werden die Hebel 111, 112 aus der in Fig. 9 mit gestrichelter Linie dargestellten Position nach innen verschwenkt, wie das durch Pfeile 141 bzw. 142 dargestellt ist. Dabei bewegt sich der Zusammenbau aus Drückerhülse 120 und Betätigungselement 122 nach proximal, wie das durch einen Pfeil 143 dargestellt ist.

Diese Wegstrecke entspricht dem zuvor beschriebenen zweiten Abschnitt des Wirkens der Vorrichtung 105. Sind die Hebel 111, 112 ganz an das Gehäuse 106 angelegt, wird die Schiebemuffe 108 über deren Stufen 129 bzw. 130 geschoben und somit vor Ausschwenken gesperrt.

Ein Boden 144 bzw. 145 jeder Längsnut 138 bzw. 140 stellt einen Anschlag dar, über den die Hebel 111, 112 nicht weiter nach innen verschoben werden können.

Auch bei der Ausführung von Fig. 9 sind entsprechend hier nicht dargestellte Spülbohrungen vorgesehen, um auch den Innenraum über einen seitlich hier nicht näher bezeichneten Stutzen mit einer Reinigungsflüssigkeit spülen zu können.

## Patentansprüche

1. Chirurgische Faß- und Haltezange, mit einem Schaft (12, 102), mit zumindest zwei Maulteilen (14, 16), die am distalen Ende des Schaftes (12, 102) angeordnet sind, mit einem Griff (18, 104) der am proximalen Ende des Schaftes (12, 102) angeordnet ist, mit einem mit dem Griff (18, 104) in Wirkverbindung stehenden Mechanismus (20) zum Öffnen und Schließen der Maulteile (14, 16), wobei der Mechanismus (20) durch Federkraft derart beaufschlagt ist, daß dadurch die Maulteile (14, 16) in Schließrichtung gedrückt werden, und durch Manipulation am Griff (18, 104) der Mechanismus gegen die Federkraft derart bewegbar ist, daß die Maulteile (14, 16) öffnen, wobei eine als starres Getriebe ausgebildete und in den Mechanismus (20) eingreifende Vorrichtung (30, 105) vorgesehen ist, mittels derer die Maulteile (14, 16) zwangsweise in eine definierte Schließendstellung (D) bewegbar sind, in der diese auf einen zwischen diesen aufgenommenen Gegenstand (90) eine vorbestimmte Haltekraft ausüben, wobei die Vorrichtung (30, 105) einen Anschlag (58, 144, 145) aufweist, der ein Überfahren der Schließendstellung (D) auch bei erhöhtem Kraftaufwand sperrt, **gekennzeichnet durch** eine Arretierung (64, 107) zum Arretieren der Maulteile (14, 16) in einer bestimmten Stellung, wobei die Arretierung (64, 107) die Maulteile (14, 16) in dieser Schließendstellung (D) arretiert.

2. Chirurgische Faß- und Haltezange nach Anspruch 1, **dadurch gekennzeichnet, daß** der Griff (18, 104) als sich in der Schaftachse längs erstreckender Griff (18, 104) ausgebildet ist, und daß der Mechanismus (20) und die Vorrichtung (30, 105) im Griff (18, 104) aufgenommen sind.

3. Chirurgische Faß- und Haltezange nach Anspruch 2, **dadurch gekennzeichnet, daß** ein Abschnitt (62) des Griffes (18) um die Schaftachse drehbar ist, wobei dieser Abschnitt (62) Teil der Vorrichtung (30) ist, und wobei die Vorrichtung (30) derart ausgestaltet ist, daß durch die Drehbewegung des Abschnitts (62) die Maulteile (14, 16) in die Schließendstellung (D) bewegbar sind.

4. Chirurgische Faß- und Haltezange nach Anspruch 3, **dadurch gekennzeichnet, daß** im Griff (18) eine Kulissenführung (34, 44) ausgebildet ist, über die die Drehbewegung des drehbaren Abschnittes (62) des Griffes (18) in eine axiale Verschiebebewegung eines Betätigungselementes (22) des Mechanismus (20) umsetzbar ist, wobei das Betätigungselement (22) mit den Maulteilen (14, 16) in Verbindung steht.

5. Chirurgische Faß- und Haltezange nach Anspruch 4, **dadurch gekennzeichnet, daß** das Betätigungselement (22) in einer Richtung gegen die Kraft einer Feder (92) längs des Schaftes (12) verschiebbar ist, und daß ein Verschieben längs dieser Richtung ein Öffnen der Maulteile (14, 16) bis in deren maximale Öffnungsstellung (A) erlaubt.

6. Chirurgische Faß- und Haltezange nach Anspruch 4 oder 5, **dadurch gekennzeichnet, daß** das Betätigungselement (22) mit einem Kulissenkörper (34) verbunden ist, der in eine Kulisse (44) eingreift, die dem drehbaren Abschnitt (62) des Griffes (18) zugehörig ist.

7. Chirurgische Faß- und Haltezange nach Anspruch 6, **dadurch gekennzeichnet, daß** die Kulisse (44) als ein hohlzylindrischer drehbarer Abschnitt (62) des Griffes ausgebildet ist, der den Kulissenkörper (34) umgibt.

8. Chirurgische Faß- und Haltezange nach Anspruch 6 oder 7, **dadurch gekennzeichnet, daß** der Kulissenkörper (34) zwei diametral gegenüberliegende, radial vorstehende Stifte (42, 43) aufweist, die je in eine umfängliche, in axialer Richtung ansteigende Nut (50, 51) der Kulisse (44) eingreifen.

9. Chirurgische Faß- und Haltezange nach Anspruch 8, **dadurch gekennzeichnet, daß** jede ansteigende Nut (50, 51) einen sich axial erstreckenden Abschnitt (52) aufweist.

10. Chirurgische Faß- und Haltezange nach Anspruch 8 oder 9, **dadurch gekennzeichnet, daß** jede Nut (50, 51) einen Endabschnitt (56) aufweist, der gegenüber dem ansteigenden Abschnitt (54) etwas in axialer Richtung abfällt.

11. Chirurgische Faß- und Haltezange nach Anspruch 10, **dadurch gekennzeichnet, daß** ein geschlossenes Ende des Endabschnittes (56) der Nut (50) den Anschlag (58) bildet.

12. Chirurgische Faß- und Haltezange nach Anspruch 11, **dadurch gekennzeichnet, daß** die Arretierung (64) zwischen einem feststehenden Griffabschnitt (40) und einem drehbaren Abschnitt (62) des Griffes (18) derart wirkt, daß die Arretierung (64) automatisch schließt, wenn die Stifte (42, 43) des Kulissenkörpers (34) den Anschlag (58) erreicht haben.

13. Chirurgische Faß- und Haltezange nach Anspruch 12, **dadurch gekennzeichnet, daß** die Arretierung (64) einen federbelasteten, sich axial erstreckenden Bolzen (66) aufweist, der, wenn sich der verdrehbare Griffabschnitt (62) in seiner Schließendstellung (D) befindet, axial sperrend verfahrbar ist, und eine Relativdrehung zwischen feststehendem Griffteil (40) und drehbarem Griffteil (62) sperrt.

14. Chirurgische Faß- und Haltezange nach Anspruch 13, **dadurch gekennzeichnet, daß** der Bolzen (66) mit einem Schiebeelement (70) verbunden ist, das von der Außenseite her bewegbar ist, jedoch der Außenkontur des Griffes (18) angepaßt ist.

15. Chirurgische Faß- und Haltezange nach einem der Ansprüche 1 bis 14, **dadurch gekennzeichnet, daß** der Griff (18) einen fest mit dem Schaft (12) verbundenen, mit diesem koaxialen zylindrischen Abschnitt (40) aufweist, auf den ein hohlzylindrischer drehbarer Abschnitt (62) aufgeschoben ist,
wobei der drehbare Abschnitt (62) und der feststehende Abschnitt (40) über eine lösbare Sicherung (76) gegen axiales Abziehen gesichert sind.

16. Chirurgische Faß- und Haltezange nach Anspruch 15, **dadurch gekennzeichnet, daß** auf dem drehbaren Abschnitt (62) eine Abschlußkappe (60) fest montiert ist.

17. Chirurgische Faß- und Haltezange nach einem der Ansprüche 1 bis 16, **dadurch gekennzeichnet, daß** zwei fassende Maulteile (14, 16) vorgesehen sind, deren Querschnittsprofil jeweils etwa halbovalförmig (88, 89) ist, und daß die geschlossenen Maulteile (14, 16) etwa ein Oval bilden.

18. Chirurgische Faß- und Haltezange nach Anspruch 17, **dadurch gekennzeichnet, daß** ein Maulteil (16) eine axial verlaufende V-Nut (86) und das andere Maulteil (14) einen in die V-Nut (86) eingreifbaren Zahn (84) aufweist.

19. Chirurgische Faß- und Haltezange nach Anspruch 1 oder 2, **dadurch gekennzeichnet, daß** die Vorrichtung (105) zumindest einen am Griff (104) angelenkten Hebel (111, 112) aufweist, dessen eines Ende (115, 116) mit dem Mechanismus (20) zum Öffnen und Schließen in Eingriff steht, und dessen anderes Ende in der Offenstellung der Maulteile (14, 16) seitlich ausgeschwenkt ist, wobei das andere Ende zum Bewegen der Maulteile (14, 16) in die Schließendstellung (D) an den Griff (104) heran verschwenkbar ist.

20. Chirurgische Faß- und Haltezange nach Anspruch 19, **dadurch gekennzeichnet, daß** der zumindest eine Hebel (111, 112) in der Schließendstellung (D) sich längs des langerstreckten Griffes (104) erstreckt.

21. Chirurgische Faß- und Haltezange nach Anspruch 20, **dadurch gekennzeichnet, daß** im Griff (104) eine Längsnut (138, 140) ausgespart ist, in die der Hebel (111, 112) in der Schließendstellung (D) der Maulteile (14, 16) passend aufnehmbar ist, und dabei die Kontur des Griffes (104) nicht überragt.

22. Chirurgische Faß- und Haltezange nach einem der Ansprüche 19 bis 21, **dadurch gekennzeichnet, daß** zwei diametral gegenüberliegend angeordnete Hebel (111, 112) vorgesehen sind.

23. Chirurgische Faß- und Haltezange nach einem der Ansprüche 19 bis 22, **dadurch gekennzeichnet, daß** die Arretierung (107) als Schiebemuffe (108) ausgebildet ist, die bei an dem Griff (104) herangeschwenkten Hebel (111, 112) über diesen schiebbar ist und ein Ausschwenken sperrt.

24. Chirurgische Faß- und Haltezange nach Anspruch 23, **dadurch gekennzeichnet, daß** die Schiebemuffe (108) in der Schließendstellung (D) der Maulteile (14, 16) über eine Kugelraste (132) arretiert ist.

25. Chirurgische Faß- und Haltezange nach einem der Ansprüche 19 bis 24, **dadurch gekennzeichnet, daß** der Mechanismus zum Öffnen und Schließen der Maulteile (14, 16) ein im Schaft (102) axial verschiebbares Betätigungselement (122) aufweist, das derart mit dem einen Ende (115, 116) des Hebels (111, 112) in Verbindung steht, daß die Verschwenkbewegung des Hebelendes (115, 116) in eine axiale Verschiebebewegung des Betätigungselementes (122) umsetzbar ist.

## Claims

1. Surgical grasping and holding forceps having a shaft (12, 102) with at least two mouth parts (14, 16) which are arranged at the distal end of the shaft (12, 102); having a grip (18, 104) which is arranged at the proximal end of the shaft (12, 102); having a mechanism (20), in working engagement with the grip (18, 104), for opening and closing the mouth parts (14, 16), the mechanism (20) being acted upon by spring force in such a way that the mouth parts (14, 16) are thereby pushed in the closing direction, and the mechanism being movable against the spring force, by manipulation at the grip (18, 104), in such a way that the mouth parts (14, 16) open; there being provided an apparatus (30, 105), configured as a rigid linkage and engaging into the mechanism (20), with which the mouth parts (14, 16) are positively movable into a defined final closed position (D) in which they exert a predefined holding force on an object (90) received between them, the apparatus (30, 105) having a stop (58, 144, 145) which prevents overshooting of the final closed position (D) even when greater force is applied, **characterized by** a lock (64, 107) for locking the mouth parts (14, 16) in a specific position, the lock (64, 107) locking the mouth parts (14, 16) in that final closed position (D).

2. Surgical grasping and holding forceps of Claim 1, **characterized in that** the grip (18, 104) is configured as a grip (18, 104) extending longitudinally in the shaft axis; and the mechanism (20) and the apparatus (30, 105) are received in the grip (18, 104).

3. Surgical grasping and holding forceps of Claim 2, **characterized in that** a segment (62) of the grip (18) is rotatable about the shaft axis, that segment (62) being part of the apparatus (30), and the apparatus (30) being configured such that the mouth parts (14, 16) are movable into the final closed position (D) by way of the rotary movement of the segment (62).

4. Surgical grasping and holding forceps of Claim 3, **characterized in that** there is configured in the grip (18) a gated guide (34, 44) by way of which the rotary movement of the rotatable segment (62) of the grip (18) can be converted into an axial displacement movement of an actuation element (22) of the mechanism (20), the actuation element (22) being connected to the mouth parts (14, 16).

5. Surgical grasping and holding forceps of Claim 4, **characterized in that** the actuation element (22) is displaceable in one direction along the shaft (12) against the force of a spring (92); and a displacement along that direction allows the mouth parts (14, 16) to open to their maximum opening position (A).

6. Surgical grasping and holding forceps of Claims 4 or 5, **characterized in that** the actuation element (22) is joined to a gate body (34) which engages into a gate (44) that belongs to the rotatable segment (62) of the grip (18).

7. Surgical grasping and holding forceps of Claim 6, **characterized in that** the gate (44) is configured as a hollow-cylindrical rotatable segment (62) of the grip which surrounds the gate body (34).

8. Surgical grasping and holding forceps of Claims 6 or 7, **characterized in that** the gate body (34) has two diametrically opposite, radially projecting pins (42, 43) which each engage into a circumferential groove (50, 51), rising in the axial direction, of the gate (44).

9. Surgical grasping and holding forceps of Claim 8, **characterized in that** each rising groove (50, 51) has an axially extending section (52).

10. Surgical grasping and holding forceps of Claims 8 or 9, **characterized in that** each groove (50, 51) has an end section (56) which slopes downward slightly in the axial direction with respect to the rising section (54).

11. Surgical grasping and holding forceps of Claim 10, **characterized in that** a closed end of the end section (56) of the groove (50) forms the stop (58).

12. Surgical grasping and holding forceps of Claim 11, **characterized in that** the lock (64) acts between a stationary grip segment (40) and the rotatable segment (62) of the grip (18) in such a way that the lock (64) automatically closes when the pins (42, 43) of the gate body (34) have reached the stop (58).

13. Surgical grasping and holding forceps of Claim 12, **characterized in that** the lock (64) has a spring-loaded axially extending stud (66) which, when the rotatable grip segment (62) is in its final closed position (D), can be moved in axially inhibiting fashion and inhibits any relative rotation between the stationary grip segment (40) and rotatable grip segment (62).

14. Surgical grasping and holding forceps of Claim 13, **characterized in that** the stud (66) is joined to a slide element (70) that is movable from the outside but is adapted to the outer contour of the grip (18).

15. Surgical grasping and holding forceps of anyone of Claims 1 through 14, **characterized in that** the grip (18) has a cylindrical segment (40), joined immovably to the shaft (12) and coaxial with it, onto which a hollow-cylindrical rotatable segment (62) is slid, the rotatable segment (62) and the stationary segment (40) being secured against axial removal via a releasable interlock (76).

16. Surgical grasping and holding forceps of Claim 15, **characterized in that** a closure cap (60) is immovably mounted on the rotatable segment (62).

17. Surgical grasping and holding forceps of anyone of Claims 1 through 16, **characterized in that** two grasping mouth parts (14, 16) are provided, the cross-sectional profile of each one being approximately semi-oval (88, 89) in shape; and the closed mouth parts (14, 16) form approximately an oval.

18. Surgical grasping and holding forceps of Claim 17, **characterized in that** one mouth part (16) has an axially extending V-groove (86) and the other mouth part (14) has a tooth (84) which can engage into the V-groove (86).

19. Surgical grasping and holding forceps of Claims 1 or 2, **characterized in that** the apparatus (105) has at least one lever (111, 112), articulated on the grip (104), whose one end (115, 116) is in engagement with the mechanism (20) for opening and closing, and whose other end is swung out laterally when the mouth parts (14, 16) are in the open position, the other end being swingable onto the grip (104) in order to move the mouth parts (14, 16) into the final closed position (D).

20. Surgical grasping and holding forceps of Claim 19, **characterized in that** in the final closed position (D) the at least one lever (111, 112) extends along the elongated grip (104).

21. Surgical grasping and holding forceps of Claim 20, **characterized in that** there is cut out from the grip (104) a longitudinal groove (138, 140) into which the lever (111, 112) can be snugly received when the mouth parts (14, 16) are in the final closed position (D) so that it does not project beyond the contour of the grip (104).

22. Surgical grasping and holding forceps of anyone of Claims 19 through 21, **characterized in that** two levers (111, 112), arranged diametrically opposite one another, are provided.

23. Surgical grasping and holding forceps of anyone of Claims 19 through 22, **characterized in that** the lock (107) is configured as a slide bushing (108) which, when the lever (111, 112) is swung onto the grip (104), can be slid over it and keeps it from swinging outward.

24. Surgical grasping and holding forceps of Claim 23, **characterized in that** the slide bushing (108) is locked by way of a ball catch (132) when the mouth parts (14, 16) are in the final closed position (D).

25. Surgical grasping and holding forceps of anyone of Claims 19 through 24, **characterized in that** the mechanism for opening and closing the mouth parts (14, 16) has an actuation element (122), axially displaceable in the shaft (102), which is connected to the one end (115, 116) of the lever (111, 112) in such a way that the pivoting movement of the lever end (115, 116) can be converted into an axial displacement movement of the actuation element (122).

## Revendications

1. Pince chirurgicale de préhension et de maintien avec une tige (12, 102), avec au moins deux parties de mâchoire (14, 16), qui sont disposées sur l'extrémité distale de la tige (12, 102), avec une poignée (18, 104) qui est disposée sur l'extrémité proximale de la tige (12, 102), avec un mécanisme (20) en liaison fonctionnelle avec la poignée (18, 104) pour l'ouverture de la fermeture des parties des mâchoires (14, 16), le mécanisme (20) étant sollicité par une force de ressort de telle sorte que les parties de mâchoires (14, 16) sont appuyées de ce fait dans le sens de fermeture, et le mécanisme pouvant être déplacé par la manipulation sur la poignée (18, 104) dans le sens contraire à la force de ressort de telle sorte que les parties de mâchoire (14, 16) s'ouvrent, et dans laquelle il est prévu un dispositif (30, 105) conçu comme une transmission rigide et s'engageant dans le mécanisme (20), au moyen duquel les parties de mâchoire (14, 16) peuvent être déplacées de façon forcée dans une position finale de fermeture (D) définie, dans laquelle ces parties exercent une force de retenue prédéfinie sur un objet (90) réceptionné entre celles-ci, le dispositif (30, 105) présentant une butée (58, 144, 145), qui bloque un dépassement de la position finale de fermeture (D) même avec une dépense de force importante, **caractérisée par** un dispositif d'arrêt (64, 107) pour le blocage des parties de mâchoire (14, 16) dans une position définie, le dispositif d'arrêt (64, 107) verrouillant les parties de mâchoire (14, 16) dans cette position finale de fermeture (D).

2. Pince chirurgicale de préhension et de maintien selon la revendication 1, **caractérisée en ce que** la poignée (18, 104) est conçue comme une poignée (18, 104) s'étendant en longueur dans l'axe de la tige, et **en ce que** le mécanisme (20) et le dispositif (30, 105) sont logés dans la poignée (18, 104).

3. Pince chirurgicale de préhension et de maintien selon la revendication 2, **caractérisée en ce qu'**une section (62) de la poignée (18) peut pivoter autour de l'axe de tige, cette section (62) faisant partie du dispositif (30), et le dispositif (30) étant conçu de telle sorte que les parties de mâchoire (14, 16) peuvent être déplacées dans la position finale de fermeture (D) par le mouvement de rotation de la section (62).

4. Pince chirurgicale de préhension et de maintien selon la revendication 3, **caractérisée en ce que** dans la poignée (18) est formé un guide de coulissement (34, 44), par lequel le mouvement de rotation de la section pivotante (62) de la poignée (18) peut être transformé en un mouvement de déplacement axial d'un élément d'actionnement (22) du mécanisme (20), l'élément d'actionnement (22) étant en liaison avec les parties de mâchoire (14, 16).

5. Pince chirurgicale de préhension et de maintien selon la revendication 4, **caractérisée en ce que** l'élément d'actionnement (22) peut être déplacé dans une direction à l'encontre de la force d'un ressort (92) le long de la tige (12) et **en ce qu'**un déplacement le long de cette direction permet une ouverture des parties de mâchoire (14, 16) jusque dans la position d'ouverture maximale (A).

6. Pince chirurgicale de préhension et de maintien selon la revendication 4 ou 5, **caractérisée en ce que** l'élément d'actionnement (22) est relié à un corps de coulisse (34), qui s'engage dans une coulisse (44), laquelle fait partie de la section pivotante (62) de la poignée (18).

7. Pince chirurgicale de préhension et de maintien selon la revendication 6, **caractérisée en ce que** la coulisse (44) est conçue comme une section (62) pivotante et en forme de cylindre creux de la poignée, qui entoure le corps de coulisse (34).

8. Pince chirurgicale de préhension et de maintien selon la revendication 6 ou 7, **caractérisée en ce que** le corps de coulisse (34) présente deux broches (42, 43) diamétralement opposées et dépassant dans le sens radial, qui s'engagent chacune dans une rainure (50, 51) périphérique et montante dans le sens axial de la coulisse (44).

9. Pince chirurgicale de préhension et de maintien selon la revendication 8, **caractérisée en ce que** chaque rainure montante (50,51) présente une section (52) s'étendant dans le sens axial.

10. Pince chirurgicale de préhension et de maintien selon la revendication 8 ou 9, **caractérisée en ce que** chaque rainure (50, 51) présente une section finale (56) qui descend légèrement dans le sens axial par rapport à la section montante (54).

11. Pince chirurgicale de préhension et de maintien selon la revendication 10, **caractérisée en ce qu'**une extrémité fermée de la section finale (56) de la rainure (50) forme la butée (58).

12. Pince chirurgicale de préhension et de maintien selon la revendication 11, **caractérisée en ce que** le dispositif d'arrêt (64) agit entre une section de poignée fixe (40) et une section pivotante (62) de la poignée (18) de telle sorte que le dispositif d'arrêt (64) ferme automatiquement lorsque les broches (42, 43) du corps de coulisse (34) ont atteint la butée (58).

13. Pince chirurgicale de préhension et de maintien selon la revendication 12, **caractérisée en ce que** le dispositif d'arrêt (64) présente un doigt (66) sollicité par un ressort et s'étendant dans le sens axial, lequel peut être déplacé en bloquant dans le sens axial lorsque la section de poignée pivotante (62) se trouve dans sa position finale de fermeture (D), et verrouille une rotation relative entre la partie de poignée (40) fixe et la partie de poignée pivotante (62).

14. Pince chirurgicale de préhension et de maintien selon la revendication 13, **caractérisée en ce que** le doigt (66) est relié à un élément coulissant (70) qui peut être déplacé à partir du côté extérieur, mais est adapté au contour extérieur de la poignée (18).

15. Pince chirurgicale de préhension et de maintien selon l'une des revendications 1 à 14, **caractérisée en ce que** la poignée (18) présente une section (40) reliée à la tige (12) et coaxiale à celle-ci, sur laquelle une section pivotante (62) en forme de cylindre creux est enfilée, la section pivotante (62) et la section fixe (40) pouvant être protégées contre un enlèvement axial par une sécurité (76) amovible.

16. Pince chirurgicale de préhension et de maintien selon la revendication 15, **caractérisée en ce qu'**un capuchon de fermeture (60) est monté de façon fixe sur la section pivotante (62).

17. Pince chirurgicale de préhension et de maintien selon l'une des revendications 1 à 16, **caractérisée en ce qu'**il est prévu deux parties de mâchoire (14, 16) de préhension, dont les profils de section présentent respectivement une forme sensiblement semi-ovale (88, 89) et **en ce que** les parties de mâchoire (14, 16) fermées forment sensiblement un ovale.

18. Pince chirurgicale de préhension et de maintien selon la revendication 17, **caractérisée en ce qu'**une partie de mâchoire (16) présente une rainure en V (86) s'étendant dans le sens axial et l'autre partie de mâchoire (14) présente une dent (84) pouvant s'engager dans la rainure en V (86).

19. Pince chirurgicale de préhension et de maintien selon la revendication 1 ou 2, **caractérisée en ce que** le dispositif (105) présente au moins un levier (111, 112) articulé sur la poignée (104), dont une extrémité (115, 116) est en prise avec le mécanisme (20) pour l'ouverture et la fermeture, et dont l'autre extrémité est basculée latéralement lorsque les parties de mâchoire (14, 16) sont dans la position ouverte, l'autre extrémité pouvant être basculée dans la position finale de fermeture (D) en se rapprochant de la poignée (104) pour le déplacement des parties de mâchoire (14, 16).

20. Pince chirurgicale de préhension et de maintien selon la revendication 19, **caractérisée en ce que** le au moins un levier (111, 112) s'étend le long de la poignée (104) étirée en longueur, dans la position finale de fermeture (D).

21. Pince chirurgicale de préhension et de maintien selon la revendication 20, **caractérisée en ce qu'**une rainure longitudinale (138, 140) est évidée dans la poignée (104), rainure dans laquelle le levier (111, 112) peut être réceptionné de façon ajustée lorsque les parties de mâchoire (14, 16) sont dans la position finale de fermeture (D), et le contour de la poignée (104) ne déborde pas dans le cas présent.

22. Pince chirurgicale de préhension et de maintien selon l'une des revendications 19 à 21, **caractérisée en ce qu'**il est prévu deux leviers (111, 112) disposés de façon diamétralement opposée.

23. Pince chirurgicale de préhension et de maintien selon l'une des revendications 19 à 22, **caractérisée en ce que** le dispositif d'arrêt (107) est conçu comme un manchon coulissant (108), lequel, lorsque le levier (111, 112) est basculé en se rapprochant de la poignée (104), peut coulisser par l'intermédiaire de ce levier et bloque un pivotement.

24. Pince chirurgicale de préhension et de maintien selon la revendication 23, **caractérisée en ce que** le manchon coulissant (108) est bloqué dans la position finale de fermeture (D) des parties de mâchoire (14, 16) au moyen d'un arrêt à bille (132).

25. Pince chirurgicale de préhension et de maintien selon l'une des revendications 19 à 24, **caractérisée en ce que** le mécanisme pour l'ouverture et la fermeture des parties de mâchoire (14, 16) présente un élément d'actionnement (122) pouvant coulisser axialement dans la tige (102), lequel est en liaison avec une extrémité (115, 116) du levier (111, 112), de telle sorte que le mouvement de basculement de l'extrémité du levier (115, 116) peut être transformé en un mouvement de déplacement axial des éléments d'actionnement (122).
